# EUROPEAN PATENT SPECIFICATION

(11) **EP 1 794 592 B1**
(45) Date of publication and mention of the grant of the patent: **18.03.2009**
(21) Application number: 05764123.5
(22) Date of filing: 30.06.2005
(51) Int. Cl.: G01N 33/558, G01N 33/569

(54) **DETECTING YEAST INFECTIONS USING A LATERAL FLOW ASSAY**
NACHWEISEN VON HEFEINFEKTIONEN UNTER VERWENDUNG EINES LATERALFLUSSTESTS
DETECTION D'INFECTIONS A LEVURES PAR DOSAGE A ECOULEMENT LATERAL

(30) Priority: 28.09.2004 US 951367
(43) Date of publication of application: 13.06.2007
(73) Proprietor: KIMBERLY-CLARK WORLDWIDE, INC., Neenah, WI 54956 (US)
(72) Inventor: WEI, Ning, Roswell, Georgia 30075 (US); YANG, Shu-Ping, Alpharetta, Georgia 30005 (US); KAYLOR, Rosann, M., Cumming, Georgia 30131 (US)
(74) Representative: Leidescher, Thomas
(86) International application number: PCT/US2005/023657
(87) International publication number: WO 2006/036247

(56) References cited:
- WO-A-00/31538
- WO-A-99/30131
- US-A1- 2003 162 236
- US-B1- 6 368 873

## Description

### Background of the Invention

The diagnosis of large pathogens like *Candida albicans*, responsible for about 1.4 million cases of yeast infections in the United States per year, is currently performed by examining samples under a microscope or by culturing a specimen. Microscopic evaluation requires a trained specialist and an instrument while culturing specimens generally requires a time of more than 24 hours to obtain results.

Flow through assays have thus far proven of limited use in detection of large pathogens because of the size of the pathogen. Various analytical procedures and devices are commonly employed in lateral flow assays to determine the presence and/or concentration of smaller analytes that may be present in a test sample. Immunoassays, for example, utilize mechanisms of the immune systems, where antibodies are produced in response to the presence of antigens that are pathogenic or foreign to the organisms. These antibodies and antigens, i.e., immunoreactants, are capable of binding with one another, thereby causing a highly specific reaction mechanism that may be used to determine the presence or concentration of that particular antigen in a biological sample. These assays require the movement of the analyte through the device, thus hindering their usefulness with larger, lower mobility, pathogens.

Despite the benefits achieved from these devices, many conventional lateral flow assays encounter significant inaccuracies when attempting to detect very large pathogens that are difficult to cause to flow. In the case of large pathogens, like, for example, *Candida albicans*, it is often the case that the complex will not properly flow to the detection zone on the membrane because of the size of the complex formed.

A need still exists, however, for an improved technique of detecting large pathogens that are difficult to cause to flow through a lateral flow device.

### Summary of the Invention

In accordance with one embodiment of the present invention, a flow-through assay device for detecting the presence or quantity of an analyte residing in a test sample is disclosed. According to the invention, the analyte is Candida alloicans. The lateral flow assay device has a porous membrane in liquid communication with a conjugate pad and a wicking pad. The conjugate pad has a gold colloid-containing detection probe. The porous membrane has a detection zone where an immobilized first capture reagent configured to bind to at least a portion of the analyte and analyte-conjugate complexes to generate a detection signal. A control zone is located downstream from the detection zone on the porous membrane and has a second capture reagent immobilized within the control zone. The conjugate pad is located upstream from the detection zone, and has detection probes with specific binding members for the analyte. A sample containing the analyte is deposited on the conjugate pad, interacts with the detection probes, and moves toward the detection zone for detection. Upstream and downstream refer to the position of an item relative to the direction of flow of a sample from the point of deposition on the assay device.

The conjugate pad contains detection probes that signal the presence of the analyte. The conjugate pad may also include other, different probe populations, including probes for indication at the control zone. The detection probes are significantly smaller than conventional probes and so may be considered nanoparticles, e.g., in the diameter range of 5 - 150 nm, more particularly the probes have a diameter between 20 and 60 nm, in order to facilitate the mobility of the probe/analyte conjugate. Suitable probes include those made from gold colloids, carbon, and dyed latex particles.

The wicking pad is in liquid communication with the membrane and provides a driving force for liquid movement due to the capillarity of the pad.

In accordance with still another embodiment, a method for detecting the presence or quantity of an analyte residing in la test sample is disclosed. According to the invention, the analyte is Candida albicans. The method comprises:
i) providing a flow-through assay device comprising a porous membrane in liquid communication with conjugate pad and a wicking pad, the porous membrane defining:
   a) a detection zone within which a first antibody is immobilized that is configured to bind to complexes formed between the analyte and the conjugated detection probes to produce a detection signal;
   b) a control zone within which is immobilized a second antibody, capable of producing a control signal when contained within the control zone; and
ii) contacting a test sample containing the analyte with the conjugated detection probes;
iii) detecting the detection signal.

### Brief Description of the Drawings

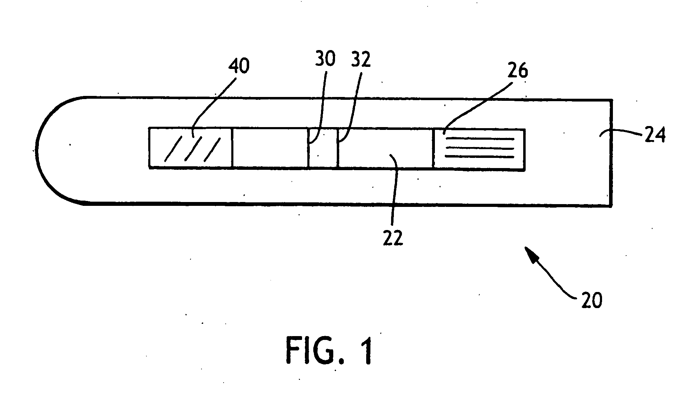

Fig. 1 is a perspective view of one embodiment of a flow-through assay device of the present invention;

### Detailed Description of Representative Embodiments

As used herein, the term "analyte" refers to Candida albicans.

As used herein, the term "test sample" generally refers to a material suspected of containing the analyte. The test sample may, for instance, include materials obtained directly from a source, as well as materials pretreated using techniques, such as, but not limited to, filtration, precipitation, dilution, lysing, distillation, mixing, concentration, inactivation of interfering components, the addition of reagents, and the like. The test sample may be derived from a biological source, such as a physiological fluid, including, blood, interstitial fluid, saliva, ocular lens fluid, cerebral spinal fluid, sweat, urine, milk, ascites fluid, mucous, synovial fluid, peritoneal fluid, vaginal fluid, menses, amniotic fluid or the like. Besides physiological fluids, other liquid samples may be used, such as water, food products, and the like. In addition, a solid material suspected of containing the analyte may also be used as the test sample.

The present invention is directed to a flow-through assay device for detecting the presence or quantity of the analyte residing in a test sample. The device utilizes multiple detection zones, one of which is premised on "control" binding of the probes and the other is premised on "sandwich" binding of the analyte. The present inventors believe that the combination of these zones may enable the detection of the analyte over extended concentration ranges. Referring to Figure 1, for instance, one embodiment of a flow-through assay device 20 that may be formed according to the present invention will now be described in more detail. As shown, the device 20 contains a porous membrane 22 optionally supported by a rigid material 24. In general, the porous membrane 22 may be made from any of a variety of materials through which the test sample is capable of passing. For example, the materials used to form the porous membrane 22 may include, but are not limited to, natural, synthetic, or naturally occurring materials that are synthetically modified, such as polysaccharides (e.g., cellulose materials such as paper and cellulose derivatives, such as cellulose acetate and nitrocellulose); polyether sulfone; polyethylene; nylon; polyvinylidene fluoride (PVDF); polyester; polypropylene; silica; inorganic materials, such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon or rayon); porous gels, such as silica gel, agarose, dextran, and gelatin; polymeric films, such as polyacrylamide; and the like. In one particular embodiment, the porous membrane 22 is formed from nitrocellulose and/or polyether sulfone materials. It should be understood that the term "nitrocellulose" refers to nitric acid esters of cellulose, which may be nitrocellulose alone, or a mixed ester of nitric acid and other acids, such as aliphatic carboxylic acids having from 1 to 7 carbon atoms.

The device 20 may also contain a wicking pad 26. The wicking pad 26 generally receives fluid that has migrated through the entire porous membrane 22. As is well known in the art, the wicking pad 26 may assist in promoting capillary action and fluid flow through the porous membrane 22.

To initiate the detection of the analyte within the test sample, a user may directly apply the test sample to a portion of the porous membrane 22 through which it may then travel in the direction illustrated by arrow "L" in Figure 1. Alternatively, the test sample may first be applied to a sample pad (not shown) that is in liquid communication with the porous membrane 22. Some suitable materials that may be used to form the sample pad include, but are not limited to, nitrocellulose, cellulose, porous polyethylene pads, and glass fiber filter paper. If desired, the sample pad may also contain one or more assay pretreatment reagents, either diffusively or non-diffusively attached thereto. In another embodiment, the test sample may be applied to the conjugate pad, such that a separate sample pad is not needed.

In the illustrated embodiment, the test sample travels from the conjugate pad 40. The conjugate pad 40 is formed from a material through which the test sample is capable of passing. For example, in one embodiment, the conjugate pad 40 is formed from glass fibers. Although only one conjugate pad 40 is shown, it should be understood that multiple conjugate pads may also be used in the present invention.

To facilitate accurate detection of the presence or absence of the analyte within the test sample, a predetermined amount of detection probes are applied at various locations of the device 20. Generally, any substance generally capable of producing a signal that is detectable visually or by an instrumental device may be used as detection probes. In the case of *Candida albicans*, however, many probes that may function with other analytes are unacceptable because the complex resulting from the analyte and probe is too large to move through the membrane to the detection zone of the device. This lack of mobility, the inventors have found, may be overcome through the use of probes made from gold colloid, carbon, or small latex particles.

These probes also may include colorimetric or fluorescent chromogens; catalysts; luminescent compounds (e.g., fluorescent, phosphorescent, etc.); radioactive compounds; hollow particles, enzymes or substrates, or organic polymer latex particles; liposomes or other vesicles containing signal producing substances; and the like. In some embodiments, the detection probes may contain a fluorescent compound that produces a detectable signal. The fluorescent compound may be a fluorescent molecule, polymer, dendrimer, particle, and the like.

The detection probes, such as described above, may be used alone or in conjunction with a particle (sometimes referred to as "beads" or "microbeads"). If small enough, naturally occurring particles such as nuclei, mycoplasma, plasmids, plastids, mammalian cells (e.g., erythrocyte ghosts), unicellular microorganisms (e.g., bacteria), polysaccharides (e.g., agarose), and the like, may be used. Latex particles that are labeled with a fluorescent or colored dye may be utilized.

Although any latex particle may be used in the present invention, the latex particles are typically formed from polystyrene, butadiene styrenes, styreneacrylic-vinyl terpolymer, polymethylmethacrylate, polyethylmethacrylate, styrene-maleic anhydride copolymer, polyvinyl acetate, polyvinylpyridine, polydivinylbenzene, polybutyleneterephthalate, acrylonitrile, vinylchloride-acrylates, and the like, or an aldehyde, carboxyl, amino, hydroxyl, or hydrazide derivative thereof. In the case of *Candida albicans* it is desired to modify the detection probes in some manner so that they are more readily able to bind to the analyte. The detection probes may be modified with certain specific binding members that are adhered thereto to form conjugated probes. Specific binding members generally refer to a member of a specific binding pair, i.e., two different molecules where one of the molecules chemically and/or physically binds to the second molecule. Immunoreactive specific binding members, for example, may include antigens, haptens, aptamers, antibodies (primary or secondary), and complexes thereof, including those formed by recombinant DNA methods or peptide synthesis.

An antibody may be a monoclonal (Mab) or polyclonal antibody (Pab), a recombinant protein or a mixture(s) or fragment(s) thereof, as well as a mixture of an antibody and other specific binding members. The details of the preparation of such antibodies and their suitability for use as specific binding members are well known to those skilled in the art. Antibodies may be conjugated with gold colloid, for example, to produce detection probes. These probes may be dried onto suitable conjugate pads and tested for sensitivity with Candida albicans solutions at varying concentrations.

Other common specific binding pairs include but are not limited to, biotin and avidin (or derivatives thereof), biotin and streptavidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Specific binding pairs may include members that are analogs of the original specific binding member; a derivative or fragment of the analyte, i.e., an analyte-analog, may be used so long as it has at least one epitope in common with the analyte.

The specific binding members may generally be attached to the detection probes using any of a variety of well-known techniques. Covalent attachment of the specific binding members to the detection probes (e.g., particles) may be accomplished using carboxylic, amino, aldehyde, bromoacetyl, iodoacetyl, thiol, epoxy and other reactive or linking functional groups, as well as residual free radicals and radical cations, through which a protein coupling reaction may be accomplished. A surface functional group may also be incorporated as a functionalized co-monomer because the surface of the detection probe may contain a relatively high surface concentration of polar groups. In addition, although detection probes are often functionalized after synthesis, in certain cases, such as poly(thiophenol), the particles are capable of direct covalent linking with a protein without the need for further modification.

The activation and/or antibody coupling may occur in a buffer, such as phosphate-buffered saline (PBS) (e.g., pH of 7.2) or 2-(N-morpholino) ethane sulfonic acid (MES) (e.g., pH of 5.3). This process forms a conjugated detection probe, where the antibody is covalently attached to the probe.

Attachment techniques other than covalent bonding, such as physical adsorption, may also be utilized in the present invention. In the case of gold colloid, proteins such as antibodies bind well to its surface through a combination of forces, including dative bonding through sulfur-gold interactions, charge attraction, and hydrophobic binding.

Referring again to Figure 1, the porous membrane 22 defines various zones configured to perform the assay. The assay device 20 also contains a detection zone 30. Although not required, the detection zone 30 is typically positioned upstream from the control zone 32. A second capture reagent is immobilized within the detection zone 30. For example, in some embodiments, the second capture reagent may be a biological capture reagent such as described above. In one embodiment, for example, the second capture reagent is an antibody specific to the analyte. The second capture reagent serves as a stationary binding site for complexes formed between the analyte and the conjugated detection probes. Specifically, analytes, typically have two or more binding sites (e.g., epitopes). Upon reaching the detection zone 30, one of these binding sites is occupied by the specific binding member of the conjugated probe. However, the free binding site of the analyte may bind to the immobilized capture reagent. Upon being bound to the immobilized capture reagent, the complexed probes form a new ternary sandwich complex.

The detection zone 30 and control zone 32 may each provide any number of distinct detection regions so that a user may better determine the concentration of a particular analyte within a test sample. Each region may contain the same capture reagents, or may contain different capture reagents. For example, the zones may include two or more distinct regions (e.g., lines, dots, etc.). The regions may be disposed in the form of lines in a direction that is substantially perpendicular to the flow of the test sample through the assay device 20. Likewise, in some embodiments, the regions may be disposed in the form of lines in a direction that is substantially parallel to the flow of the test sample through the assay device 20.

Although various embodiments of device configurations have been described above, it should be understood, that a device may generally have any configuration desired, and need not contain all of the components described above. Regardless of their particular configuration of the assay device 20, the control zone 32 and detection zone 30 function in tandem to improve the analyte detection range.

The following examples illustrate various embodiments of the invention.

### Examples

40 nm diameter gold colloid was conjugated with monoclonal and polyclonal antibodies. The monoclonal antibodies were Mab1 (catalog number 10-C07, from Fitzgerald Industries International, Inc. of Concord, MA 01742-3049 USA) and the polyclonal antibodies were Pab2 (catalog number B65411R from Biodesign International-of Saco, Maine USA 04072). On nitrocellulose membrane, (either HF075 and HF120 from Millipore Corporation of Billerica, MA.,USA) was striped with antibody Pab2 at 1 mg/ml as the capture reagent and goat anti-mouse at 1 mg/ml as control line. The membrane was dried at about 37 C for about 1 hour. After the membrane was taken out from the oven, the wicking pad (Millipore Corporation) was laminated to the upper portion near the control line. The card was cut into 4 mm wide half strips and immersed into 96 well-plate for testing. Each of the conjugate was tested against a buffer control and Candida albicans solution. When the testing well contains buffer solution, the capture line was negative, only the control line turned positive. When the testing wells contain Candida albicans, both the capture and control lines turned positive. The system was able to detect 10⁷ - 10⁸ CFU/ml with these two antibody pairs.

Additional testing was performed with two more conjugates, Mab3 (catalog number 1750-5007, from Biogenesis Inc. of Brentwood, NH 03833), Mab5 (catalog number A63100069P, from BioSpacific Corporation of Emeryville, CA 94608), using the same Pab2 as the capture reagent. The conjugates were used to test the half sticks with buffer and Candida albicans solutions. Again, the system indicated negative for the buffer control while turned positive with a Candida albicans stock solution (10⁸ CFU/ml) and 10X diluted (10⁷ CFU/ml) solutions.

While the positive tests with the organism solutions, the tests were also positive with the spun down Candida albicans which were re-suspended in a different buffer, indicating the positive test for the Candida albicans may be due to the whole organism, debris or the secreted proteins from the whole organism.

## Claims

1. A lateral flow assay device for detecting the presence or quantity of an analyte residing in a test sample, said lateral flow assay device comprising a porous membrane in liquid communication with a conjugate pad and a wicking pad:
said conjugate pad located upstream from a detection zone, said conjugate pad having detection probes with specific binding members for the analyte comprising nanoparticles and;
said detection zone having an immobilized first capture reagent, said first capture reagent being configured to bind to at least a portion of said analyte and analyte-conjugate complexes to generate a detection signal having an intensity;
a control zone located downstream from said detection zone, wherein a second capture reagent is immobilized within said control zone, said second capture reagent being configured to bind to said conjugate or conjugate-analyte complexes;
wherein a sample containing said analyte is deposited on said conjugate pad and said sample moves toward said control zone,
wherein said analyte is Candida albicans.

2. A lateral flow assay device as defined in claim 1, wherein said conjugated detection probes comprise a substance selected from the group consisting of chromogens, catalysts, luminescent compounds, radioactive compounds, visual labels, liposomes, and combinations thereof.

3. A lateral flow assay device as defined in claim 1, wherein said conjugated detection probes comprise a luminescent compound.

4. A lateral flow assay device as defined in claim 1, wherein said conjugated detection probes comprise a visual label.

5. A lateral flow assay device as defined in claim 1, wherein said specific binding member is selected from the group consisting of antigens, haptens, aptamers, primary or secondary antibodies, biotin, and combinations thereof.

6. A lateral flow assay device as defined in claim 1, wherein said first capture reagent is selected from the group consisting of antigens, haptens, protein A or G, neutravidin, avidin, streptavidin, captavidin, primary or secondary antibodies, and complexes thereof.

7. A lateral flow assay device as defined in claim 1, wherein said second capture reagent is selected from the group consisting of antigens, haptens, protein A or G, neutravidin, avidin, streptavidin, captavidin, primary or secondary antibodies, and complexes thereof.

8. A method for detecting the presence or quantity of an analyte residing in a test sample, said method comprising:
i) providing a lateral flow assay device comprising a porous membrane, in liquid communication with a conjugate pad and a wicking pad, said conjugate pad having nanoparticle detection probes conjugated with a specific binding member for the analyte, said porous membrane defining a detection zone in which a first capture reagent is immobilized and a control zone within which a second capture reagent is immobilized, wherein said control zone is located downstream from said detection zone;
ii) contacting said test sample containing the analyte with the conjugate pad;
iii) detecting a detection signal,
wherein said analyte is Candida albicans.

9. A method as defined in claim 8, wherein said conjugated detection probes comprise a substance selected from the group consisting of chromogens, catalysts, luminescent compounds, radioactive compounds, visual labels, liposomes, and combinations thereof.

10. A method as defined in claim 8, wherein said conjugated detection probes comprise a visual label.

11. A method as defined in claim 8, wherein said specific binding member is selected from the group consisting of antigens, haptens, aptamers, primary or secondary antibodies, biotin, and combinations thereof.

12. A method as defined in claim 8, wherein said first capture reagent is selected from the group consisting of antigens, haptens, protein A or G, neutravidin, avidin, streptavidin, captavidin, primary or secondary antibodies, and complexes thereof.

13. A method as defined in claim 8, wherein said second capture reagent is selected from the group consisting of antigens, haptens, protein A or G, neutravidin, avidin, streptavidin, captavidin, primary or secondary antibodies, and complexes thereof.

14. A method as defined in claim 8, wherein said second capture reagent comprises a polyelectrolyte.

## Patentansprüche

1. Lateral-Flow-Assay-Vorrichtung zum Detektieren der Gegenwart oder Quantität eines in einer Testprobe vorhandenen Analyten, wobei die Lateral-Flow-Assay-Vorrichtung eine poröse Membran in Flüssigkommunikation mit einem Konjugat-Pad und einem Saug-Pad umfasst:
wobei das Konjugat-Pad stromaufwärts von einer Detektionszone angeordnet ist, und das Konjugat-Pad Detektionssonden, umfassend Nanopartikel, mit spezifischen Bindeelementen für den Analyten umfasst, und;
in der Detektionszone ein erstes Abfangreagens immobilisiert ist, wobei das erste Abfangreagens derart konfiguriert ist, so dass es an mindestens einen Teil des Analyten und von Analyt-Konjugat-Komplexen bindet, um ein Detektionssignal mit einer Intensität zu erzeugen;
wobei eine Kontrollzone stromabwärts von der Detektionszone angeordnet ist, wobei in der Kontrollzone ein zweites Abfangreagens immobilisiert ist, und das zweite Abfangreagens derart konfiguriert ist, so dass es an das Konjugat oder an Konjugat-Analyt-Komplexe bindet;
wobei eine den Analyten enthaltende Probe auf das Konjugat-Pad aufgetragen wird und die Probe in Richtung der Kontrollzone wandert,
wobei der Analyt Candida albicans ist.

2. Lateral-Flow-Assay-Vorrichtung nach Anspruch1, wobei die konjugierten Detektionssonden eine Substanz umfassen, ausgewählt aus der Gruppe, bestehend aus Chromogenen, Katalysatoren, lumineszierenden Verbindungen, radioaktiven Verbindungen, sichtbaren Markierungen, Liposomen, und Kombinationen davon.

3. Lateral-Flow-Assay-Vorrichtung nach Anspruch1, wobei die konjugierten Detektionssonden eine lumineszierende Verbindung umfassen.

4. Lateral-Flow-Assay-Vorrichtung nach Anspruch1, wobei die konjugierten Detektionssonden eine sichtbare Markierung umfassen.

5. Lateral-Flow-Assay-Vorrichtung nach Anspruch1, wobei das spezifische Bindeelement ausgewählt ist aus der Gruppe, bestehend aus Antigenen, Haptenen, Aptameren, primären oder sekundären Antikörpern, Biotin, und Kombinationen davon.

6. Lateral-Flow-Assay-Vorrichtung nach Anspruch1, wobei das erste Abfangreagens ausgewählt ist aus der Gruppe, bestehend aus Antigenen, Haptenen, Protein A oder G, Neutravidin, Avidin, Streptavidin, Captavidin, primären oder sekundären Antikörpern, und Komplexen davon.

7. Lateral-Flow-Assay-Vorrichtung nach Anspruch1, wobei das zweite Abfangreagens ausgewählt ist aus der Gruppe, bestehend aus Antigenen, Haptenen, Protein A oder G, Neutravidin, Avidin, Streptavidin, Captavidin, primären oder sekundären Antikörpern, und Komplexen davon.

8. Verfahren zum Detektieren der Gegenwart oder Quantität eines in einer Testprobe vorhandenen Analyten, wobei das Verfahren umfasst:
i) Bereitstellen einer Lateral-Flow-Assay-Vorrichtung, umfassend eine poröse Membran in Flüssigkommunikation mit einem Konjugat-Pad und einem Saug-Pad, wobei das Konjugat-Pad Nanopartikel-Detektionssonden, konjugiert mit einem spezifischen Bindeelement für den Analyten umfasst, die poröse Membran eine Detektionszone definiert, in der ein erstes Abfangreagens immobilisiert ist, und eine Kontrollzone, in der ein zweites Abfangreagens immobilisiert ist, wobei die Kontrollzone stromabwärts von der Detektionszone angeordnet ist;
ii) In Kontakt Bringen der den Analyten enthaltenden Testprobe mit dem Konjugat-Pad;
iii) Detektieren eines Detektionssignals,
wobei der Analyt Candida albicans ist.

9. Verfahren nach Anspruch 8, wobei die konjugierten Detektionssonden eine Substanz umfassen, ausgewählt aus der Gruppe, bestehend aus Chromogenen, Katalysatoren, lumineszierenden Verbindungen, radioaktiven Verbindungen, sichtbaren Markierungen, Liposomen, und Kombinationen davon.

10. Verfahren nach Anspruch 8, wobei die konjugierten Detektionssonden eine sichtbare Markierung umfassen.

11. Verfahren nach Anspruch 8, wobei das spezifische Bindeelement ausgewählt ist aus der Gruppe, bestehend aus Antigenen, Haptenen, Aptameren, primären oder sekundären Antikörpern, Biotin, und Kombinationen davon.

12. Verfahren nach Anspruch 8, wobei das erste Abfangreagens ausgewählt ist aus der Gruppe, bestehend aus Antigenen, Haptenen, Protein A oder G, Neutravidin, Avidin, Streptavidin, Captavidin, primären oder sekundären Antikörpern, und Komplexen davon.

13. Verfahren nach Anspruch 8, wobei das zweite Abfangreagens ausgewählt ist aus der Gruppe, bestehend aus Antigenen, Haptenen, Protein A oder G, Neutravidin, Avidin, Streptavidin, Captavidin, primären oder sekundären Antikörpern, und Komplexen davon.

14. Verfahren nach Anspruch 8, wobei das zweite Abfangreagens einen Polyelektrolyten umfasst.

## Revendications

1. Dispositif pour dosage à écoulement latéral pour détecter la présence ou la quantité d'un analyte se trouvant dans un échantillon à tester, ledit dispositif pour dosage à écoulement latéral comprenant une membrane poreuse en communication de liquide avec un tampon de conjugué et un tampon de méchage ;
ledit tampon de conjugué étant situé en amont d'une zone de détection, ledit tampon de conjugué ayant des sondes de détection pourvues d'éléments de liaison spécifiques à l'analyte comprenant des nanoparticules ; et
ladite zone de détection comportant un premier réactif de capture immobilisé, ledit premier réactif de capture étant configuré pour se lier à au moins une portion dudit analyte et des complexes analyte-conjugué pour générer un signal de détection ayant une intensité ;
une zone de contrôle située en aval de ladite zone de détection, un second réactif de capture étant immobilisé au sein de ladite zone de contrôle, ledit second réactif de capture étant configuré pour se lier audit conjugué ou aux complexes conjugué-analyte ;
un échantillon contenant ledit analyte étant déposé sur ledit tampon de conjugué et ledit échantillon se déplaçant en direction de ladite zone de contrôle ;
ledit analyte étant Candida albicans.

2. Dispositif pour dosage à écoulement latéral selon la revendication 1, dans lequel lesdites sondes de détection conjuguées comprennent une substance sélectionnée dans le groupe consistant en les chromogènes, les catalyseurs, les composés luminescents, les composés radioactifs, les marqueurs visuels, les liposomes, et leurs combinaisons.

3. Dispositif pour dosage à écoulement latéral selon la revendication 1, dans lequel lesdites sondes de détection conjuguées comprennent un composé luminescent.

4. Dispositif pour dosage à écoulement latéral selon la revendication 1, dans lequel lesdites sondes de détection conjuguées comprennent un marqueur visuel.

5. Dispositif pour dosage à écoulement latéral selon la revendication 1, dans lequel ledit élément de liaison spécifique est sélectionné dans le groupe consistant en les antigènes, les haptènes, les aptamères, les anticorps primaires et secondaires, la biotine, et leurs combinaisons.

6. Dispositif pour dosage à écoulement latéral selon la revendication 1, dans lequel ledit premier réactif de capture est sélectionné dans le groupe consistant en les antigènes, les haptènes, la protéine A ou G, la neutravidine, l'avidine, la streptavidine, la captavidine, les anticorps primaires ou secondaires, et leurs complexes.

7. Dispositif pour dosage à écoulement latéral selon la revendication 1, dans lequel ledit second réactif de capture est sélectionné dans le groupe consistant en les antigènes, les haptènes, la protéine A ou G, la neutravidine, l'avidine, la streptavidine, la captavidine, les anticorps primaires ou secondaires, et leurs complexes.

8. Procédé de détection de la présence ou de la quantité d'un analyte se trouvant dans un échantillon à tester, ledit procédé comprenant :
i) la fourniture d'un dispositif pour dosage à écoulement latéral comprenant une membrane poreuse, en communication de liquide avec un tampon de conjugué et un tampon de méchage, ledit tampon de conjugué ayant des sondes de détection sur nanoparticules conjuguées à un élément de liaison spécifique à l'analyte, ladite membrane poreuse définissant une zone de détection dans laquelle est immobilisé un premier réactif de capture, et une zone de contrôle dans laquelle est immobilisé un second réactif de capture, ladite zone de contrôle étant située en aval de ladite zone de détection ;
ii) la mise en contact dudit échantillon à tester, contenant l'analyte, avec le tampon de conjugué ;
iii) la détection d'un signal de détection,
ledit analyte étant Candida albicans.

9. Procédé selon la revendication 8, dans lequel lesdites sondes de détection conjuguées comprennent une substance sélectionnée dans le groupe consistant en les chromogènes, les catalyseurs, les composés luminescents, les composés radioactifs, les marqueurs visuels, les liposomes, et leurs combinaisons.

10. Procédé selon la revendication 8, dans lequel lesdites sondes de détection conjuguées comprennent un marqueur visuel.

11. Procédé selon la revendication 8, dans lequel ledit élément de liaison spécifique est sélectionné dans le groupe consistant en les antigènes, les haptènes, les aptamères, les anticorps primaires et secondaires, la biotine, et leurs combinaisons.

12. Procédé selon la revendication 8, dans lequel ledit premier réactif de capture est sélectionné dans le groupe consistant en les antigènes, les haptènes, la protéine A ou G, la neutravidine, l'avidine, la streptavidine, la captavidine, les anticorps primaires ou secondaires, et leurs complexes.

13. Procédé selon la revendication 8, dans lequel ledit second réactif de capture est sélectionné dans le groupe consistant en les antigènes, les haptènes, la protéine A ou G, la neutravidine, l'avidine, la streptavidine, la captavidine, les anticorps primaires ou secondaires, et leurs complexes.

14. Procédé selon la revendication 8, dans lequel ledit second réactif de capture comprend un polyélectrolyte.
